Europäisches Patentamt

(19) European Patent Office

Office européen des brevets

(11) Publication number: 0 109 531
A1

(12) # EUROPEAN PATENT APPLICATION

(21) Application number: 83110163.9

(22) Date of filing: 12.10.83

(51) Int. Cl.³: A 61 M 1/03

(30) Priority: 16.11.82 SE 8206520
25.11.82 SE 8206708

(43) Date of publication of application:
30.05.84 Bulletin 84/22

(84) Designated Contracting States:
AT BE CH DE FR GB IT LI LU NL

(71) Applicant: Gambro Lundia AB
Box 10101
S-220 10 Lund(SE)

(72) Inventor: Freiburghaus, Christian Auguste
Brynjevägen 46
S-240 21 Löddeköpinge(SE)

(74) Representative: Boberg, Nils Gunnar Erik et al,
Gambro AB Patent Department Box 10101
S-220 10 Lund(SE)

(54) A column and a system for the removal of specific antibodies from a body fluid.

(57) A column (1) is intended to be used in an extracorporeal system for the removal of antibodies from a body fluid. The column is filled with, for example, porous gel particles (20) upon which antigens with the capacity of specifically binding the antibodies are immobilized.

The characteristic feature of the column is that the antigens are immobilized or distributed in such a manner that the mutual distance between them (or rather their active binding sites) in the main is greater than the distance between the two Fab-parts of the antibody.

The column and the system are intended in particular for the removal of antibodies against factor IX from blood of a patient with haemophilia B, the antigen in this case being factor IX.

Fig.2

EP 0 109 531 A1

TITLE

A COLUMN AND A SYSTEM FOR THE REMOVAL OF SPECIFIC ANTIBODIES

FROM A BODY FLUID


TECHNICAL FIELD

The invention relates to a column intended to be used in an extracorporeal system for the removal of specific antibodies from a body fluid, the column comprising antigens immobilized on a carrier which are directed specifically at the said antibodies.

The invention also relates to an extracorporeal system for the abovementioned purpose and comprising a column of the type mentioned, the system comprising moreover means for the feed and withdrawal respectively of the said body fluid.

In particular, though without being limited to this, the invention relates to a column and a system for the removal of antibodies against factor IX of a patient with haemophilia B and with antibodies against factor IX at high titre ($>3$ units antibodies against factor IX per ml plasma corresponding to $>9$ Bethesda units per ml plasma).

The expression "body fluid" here and in the following relates to blood, plasma or similar blood fluids, even if it is used in what follows first and foremost to mean blood.


BACKGROUND ART

In, for example, the journal Blood, vol.58, no.1 (July), 1981, pages 38-44, a method is described for the removal of antibodies against factor IX in a patient with haemophilia B, the blood which has been withdrawn from the patient first being separated into a plasma fraction and a blood-cell fraction, and the separated plasma fraction subsequently made to flow through a column in contact with protein A bound up to a Sepharose gel for the adsorption of the antibodies.

Although the method described certainly produces the intended therapeutic effect insofar as adsorption of the actual antibodies against factor IX is concerned, it nevertheless has deficiencies which make it not wholly satisfactory.

For example, it is unspecific inasmuch as not only the actual antibodies are adsorbed, but also the bulk of the immunoglobulins present capable of binding to protein A. (cf. page 42, righthand column, line 19-21). Moreover it is necessary, as described in the article, first to separate the blood-cells so as to avoid contact between these and protein A.

Against this background it is the object of the invention to provide an improvement of the method described, making it possible to treat blood directly without previous separation of blood-cells and allowing the adsorption of the actual antibodies to be carried out with a high degree of selectivity.

This object is achieved in accordance with the invention with the help of the column and the system which are defined in the claims and which will be described in more detail in the following.


DISCLOSURE OF INVENTION

In accordance with the invention thus a column is provided intended to be used in an extracorporeal system for the removal of specific antibodies from a body fluid. The column comprises antigens immobilized on a carrier which are directed specifically at the said antibodies, and is characterized in that the antigens are immobilized so that the mutual distance between their active binding sites in the main is greater than the distance between the two Fab-regions of the actual antibody.

Furthermore an extracorporeal system for the removal of specific antibodies from a body fluid is provided. The system comprises a column which contains antigens immobilized on a carrier, which are directed specifically at the said antibodies together with means for the feed and withdrawal respectively of the said body fluid, and is characterized in that the said antigens are immobilized so that the mutual distance between their active binding sites in the main is greater than the distance between the two Fab-regions of the actual antibody.

As a result of the immobilization of the antigens in the manner indicated above it has been found that after completed

- 3 -

treatment the bound up antibodies can be set free and eluated very readily and with nearly 100% efficiency.  The column in accordance with the invention is very suitable, therefore, for regeneration and reutilization as many times as may be desired.

A conceivable theory to explain the regenerative capacity of the column obtained in accordance with the invention could be that the antibodies which can bind with both their Fab parts, to an increasing extent bind with only one of their Fab-parts at the rate as the distance between the immobilized antigens increases.  In this manner the binding strength between the antibodies and the antigens would decrease to a corresponding extent, with the consequence that it becomes easier for the bound antibodies to be set free.

On the other hand, if the distance between the antigens diminishes, that is to say if the antigens sit more closely together, the possibility of the antibodies binding with both their Fab-parts increases, which means that the binding strength between the antibodies and the antigens increases and it becomes more difficult, consequently, for the bound antibodies to be set free.

A logical conclusion to be drawn from the above reasoning is, therefore, that optimum binding capacity and regenerative capacity are achieved when the distance between the immobilized antigens (or rather the active binding sites of the antigens) narrowly exceeds the distance between the two Fab-parts of the actual antibodies.  Theoretically in this situation the greatest possible amount of antigen (binding capacity) should be able to be immobilized on the carrier on condition that the actual antibodies should be able to bind only with one Fab-part (corresponding to high regenerating capacity).

BRIEF DESCRIPTION OF DRAWINGS

In the following the invention will be described in more detail with reference to the enclosed drawings, wherein

Fig.1 illustrates graphically the observed connection between regenerative capacity and antigen density on the column in

- 4 -

accordance with the invention, and

Fig.2 shows schematically the set-up of an extracorporeal system in accordance with the invention comprising a column of the characteristics illustrated in fig.1.

## BEST MODE OF CARRYING OUT THE INVENTION

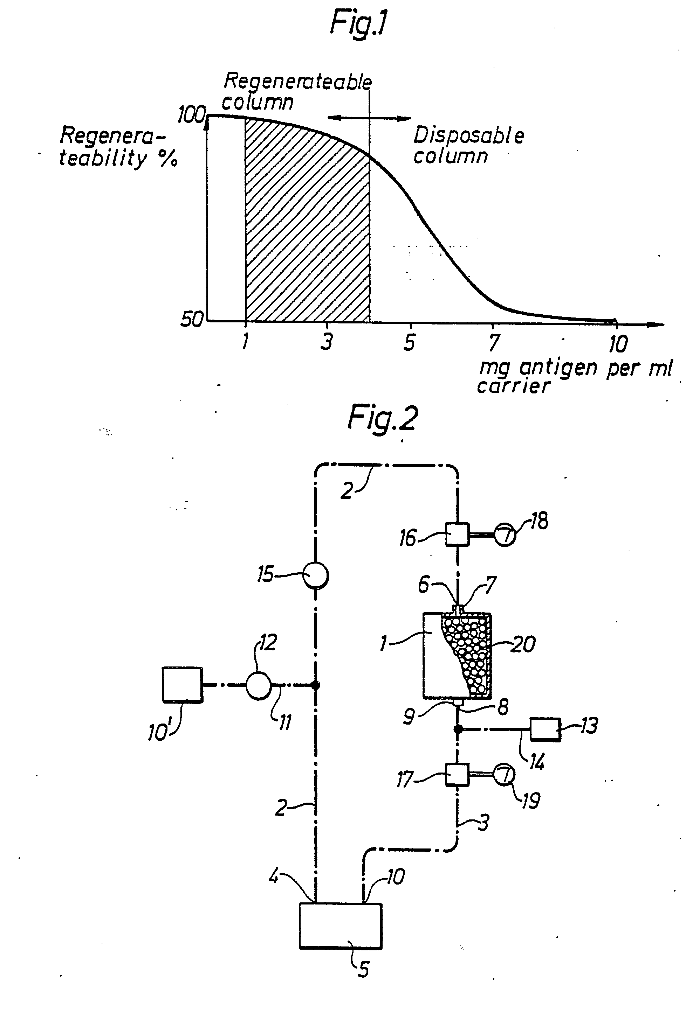

As is evident from fig.2, the system in accordance with the invention comprises a column 1 and a line 2 for the feed of body fluid, for example blood, and a line 3 for the withdrawal of body fluid. The line 2 can be, or is, connected with its one end 4 to a source 5 of body fluid and with its other end 6 it is connected to the corresponding inlet port 7 of the column 1. In a corresponding manner the line 3 is connected with one end 8 to an outlet port 9 of the column 1 and with its other end 10 it can be, or is, connected to the source 5 of body fluid.

When the system in accordance with the invention is intended to be used, for example, in conjunction with the treatment of a patient's blood (corresponding to source 5) it preferably comprises means for the feed of an anticoagulant, e.g. an ACD solution, in the form of a container 10 which holds the anticoagulant and is connected to the line 2 via a connection line 11 comprising a pump 12 for the appropriate metering of the anticoagulant. In the event of the anticoagulant consisting of an ACD solution, the system preferably also includes means for the feed of a neutralizing agent, e.g. a calcium solution, in the form of a container 13 which holds the neutralizing agent and is connected to the line 3 via a branch line 14. As is customary in conjunction with the extracorporeal treatment of bood in accordance with the principle which is described here, the system shown moreover includes a pump 15 and air controllers 16 and 17 as well as pressure gauges 18 and 19. Such components, as mentioned earlier, are generally known in this field and hardly need to be described in any great detail therefore in the present context.

As described above, the column 1 in accordance with the invention comprises antigens which are directed specifically at the antibodies which are to be removed from the body fluid. The antigens are in fact immobilized on a carrier 20 in such a manner that the mutual distance between the active binding sites of the immobilized antigens in the main is greater than the distance between the two Fab-regions of the actual antibody.

Since the maximum distance between the two Fab-regions of an antibody normally amounts to approx. 140 $\overset{o}{A}$, this means that the immobilized antigens in the column 1 are situated at a distance from one another of at least 140 $\overset{o}{A}$. Converted into an amount of immobilized antigen per ml of carrier such a distribution of antigens corresponds to a value of less than approx. 4mg. antigen/ml carrier. Even though it is certainly possible to bind a greater quantity of antigen to the carrier than the upper limit specified here and nevertheless achieve an intended adsorption of antibodies, it is appropriate with a view to the regenerative capacity of the column not to use such a large amount. This position has already been referred to in the introduction, but will be explained in more detail in the following in connection with fig.1.

It is particularly advantageous in accordance with the invention to use porous macromolecular gel particles as a carrier for the immobilized antigens, since such gel particles present suitable flow conditions for the body fluid and make it possible in fact to carry out the intended treatment directly on blood.

Such macromolecular carriers in accordance with the invention can be appropriately gel particles containing agarose (Pharmacia Fine Chemicals AB, Upsala, Sweden). Gel particles containing agarose of the type Sepharose 6 MB of a particle size of 200 - 300 μm have been found to be particularly suitable.

The invention is not limited to the choice of any one antigen, but it has been found to be particularly apt in connection with factor IX for the removal of antibodies against factor IX in a patient with haemophilia B and antibodies against factor IX. In this case immobilized factor IX is used in such an amount that it can bind maximally between 2 and 10 mg antibody per ml carrier, preferably 7 mg antibody per ml carrier. Converted to the amount immobilized pure factor IX per ml carrier this corresponds to approx. 1 - 4 mg and 3 mg respectively of pure factor IX per ml carrier.

The antigens can be immobilized in a manner known in itself on the carrier, for example, by allowing a solution containing the antigen to come into contact with the carrier in any suitable manner. This may be achieved, for example, in that the solution is made to flow through a column packed with the carrier or by suspending the carrier in the antigen solution.

If, for example, factor IX is to be bound to a carrier of Sepharose gel particles in accordance with the invention this may be done by causing a solution containing factor IX to pass through a column packed with the gel particles pre-activated with BrCN.

In fig.1 is shown graphically the connection between regenerative capacity and antigen density of the column in accordance with the invention. As is evident from the hatched area in fig.1, the column possesses a nearly 100% regenerative capacity within the range of antigen density of 1 - 4 mg antigen per ml carrier. When the antigen densities rise above this range, the regenerative capacity of the column diminishes. This means, in accordance with the invention, that at these higher antigen densities the column has a more or less limited number of reutilization possibilities and is described, therefore, as a "single-pass column". In the case of antigen densities below approx. 4 mg/ml carrier, on the other hand, the column has an almost unlimited number (at least 50) of reutilization facilities and may be described, therefore, as a "regenerable column". The optimum antigen density of a column in accordance with the invention, as mentioned earlier, is approx. 3 ml antigen per ml carrier.

If, for example, a patient with haemophilia B and with antibodies against factor IX is to be treated, the system, or rather the column, in accordance with the invention operates preferably as follows: Blood is withdrawn from the patient 5 through the line 2 and is pumped with the help of the pump 15 (approx. 5 - 25 ml/min) into the column 1. In the column 1 the antibodies against factor IX are collected with the help of factor IX which is immobilized on a carrier of, for example, Sepharose 6 MB

0109531

- 7 -

gel particles.  The blood so treated is returned subsequently
to the patient 5 through the line 3 via the air controller 17
and pressure gauge 19.  Anticoagulant, for example citrate
solution (ACD), is added in a suitable amount (1:8 - 1:19) to
the blood in line 2 from the container 10 through connection
line 11 with the help of the pump 12.  Similarly a neutralizing
agent, e.g. calcium solution, is added to the blood treated in
line 3 from container 13 through branch line 14.

## INDUSTRIAL APPLICABILITY

The column and the system in accordance with the invention
are applicable under circumstances where it is desirable to
remove specific antibodies from a body fluid, and especially in
conjunction with the treatment of a patient with haemophilia B
and antibodies against factor IX at a high titre.

0109531

- 8 -

CLAIMS

1. A column intended to be used in an extracorporeal system for the removal of specific antibodies from a body fluid, the column (1) comprising antigens immobilized on a carrier (20)which are directed specifically at the said antibodies, c h a r a c t e r i z e d  in that the said antigens are immobilized so that the mutual distance between their active binding sites in the main is greater than the distance between the Fab-regions of the actual antibody.

2. A column in accordance with claim 1, c h a r a c t e r i z e d in that the said antigens are immobilized on a carrier in the form of porous macromolecular gel particles.

3. A column in accordance with claim 1 or 2, c h a r a c t e r - i z e d  in that the said carrier is chosen among gel particles containing agarose, in particular, Sepharose.

4. A column in accordance with anyone of the preceding claims, c h a r a c t e r i z e d  in that the immobilized antigen is factor IX.

5. A column in accordance with anyone of the preceding claims, c h a r a c t e r i z e d  in that the immobilized antigen is used in such an amount that it can bind maximally between 2 and 10 mg antibody per ml gel, preferably approx. 7 mg antibody per ml gel.

6. A system for the removal of specific antibodies from a body fluid which comprises a column (1) containing antigens immobilized on a carrier (20) which are directed specifically at the said antibodies together with means for the feed and withdrawal respectively of the said body fluid, c h a r a c t e r i z e d  in that the said antigens are immobilized so that the mutual distance between their active binding sites in the main is greater than the distance between the Fab-regions of the actual antibody.

7. A system in accordance with claim 6, c h a r a c t e r i z e d in that the immobilized antigen is used in such an amount that it can bind maximally between 2 and 10 mg antibody per ml gel, preferably approx. 7 mg antibody per ml gel.

8. A system in accordance with claim 6 or 7, c h a r a c t e r -

i z e d    in that the said means for the feed of body fluid comprise a line (2) whose one end (6) is connected to an inlet port (7) of the column (1) and whose other end (4) can be connected to a source (5) of the said body fluid, and that the said means for the withdrawal of body fluid comprise a line (3) whose one end (8) is connected to an outlet port (9) of the column (1) and whose other end (10) can be connected to the said source (5) of body fluid.

9.    A system in accordance with anyone of claims 6 - 8, c h a r a c t e r i z e d    in that the said means for the feed of body fluid comprise means (10,11,12) for the feed of an anticoagulant, for example ACD-solution.

10.    A system in accordance with claim 9, c h a r a c t e r - i z e d    in that the said means for the withdrawal of body fluid comprise means (13,14) for the feed of a substance neutralizing the anticoagulant, for example, a calcium solution.

11.   Application of the column in accordance with anyone of the claims 1 - 5 for the collecting of antibodies which had been bound to the said immobilized antigens.

**Fig.1**

**Fig.2**

# EUROPEAN SEARCH REPORT

Application number

EP 83 11 0163

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | FR-A-2 355 515 (MITSUI)<br><br>* Whole document; figure 1; page 3, lines 9-10; page 5, lines 3-4,11-15 * | 1-3,5-7,9,11 | A 61 M 1/03 |
| Y | AFFINITY CHROMATOGRAPHY, PRINCIPLES & METHODS, 1971, Pharmacia Fine Chemicals, Upsala, SE, pages 7,14-17<br>* Page 16, lines 21-23,31-33 * | 1-3,5-7,9,11 | |
| Y | DE-A-2 029 760 (WELLCOME)<br><br>* Claim 1; page 11, lines 11-18 * | 1-3,5-7,9,11 | |
| A | EP-A-0 046 470 (STOLLE)<br>* Whole document; page 9, line 7 * | 4 | TECHNICAL FIELDS SEARCHED (Int. Cl. ³) |
| L | F.W. PUTNAM: "The plasma proteins", 2nd edition, vol. 1, 1975, pages 115-116, Academic Press, New York, US<br>* Table I * | | A 61 M<br>A 61 K |

--- -/-

The present search report has been drawn up for all claims

| Place of search | Date of completion of the search | Examiner |
|---|---|---|
| THE HAGUE | 14-02-1984 | PETZUCH M. |

European Patent Office

**EUROPEAN SEARCH REPORT**

| | DOCUMENTS CONSIDERED TO BE RELEVANT | | Page 2 |
| --- | --- | --- | --- |
| Category | Citation of document with indication, where appropriate, of relevant passages | Relevant to claim | CLASSIFICATION OF THE APPLICATION (Int. Cl. ³) |
| L | FEDERATION OF EUROPEAN BIOCHEMICAL SOCIETIES, eighth meeting, 1972, vol. 26 "Immunoglobulins: cell bound receptors and humoral antibodies", pages 61-67, North-Holland/American Elsevier, Amsterdam, NL E. HABER et al.: "The three dimensional structure of immunoglobulins" * Page 65 * | | |
| A | WO-A-8 203 331 (BIOLOGICAL & INTERFERON PRODUCTS) * Figure 1 * | 8 | |
| A | US-A-4 300 551 (KINNEY) * Figure 1, reference sign 31; column 3, lines 4-10 * | 10 | |
| | | | TECHNICAL FIELDS SEARCHED (Int. Cl. ³) |

The present search report has been drawn up for all claims

| Place of search | Date of completion of the search | Examiner |
| --- | --- | --- |
| THE HAGUE | 14-02-1984 | PETZUCH M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03 82